# EUROPEAN PATENT APPLICATION

(11) **EP 0 857 715 A1**
(43) Date of publication of application: **12.08.1998**
(21) Application number: 98200169.5
(22) Date of filing: 22.01.1998
(51) Int. Cl.: C07C 67/03, C07C 69/76

(54) **Recovery of monomers from polyethylene naphthalate using low pressure methanolysis**

(30) Priority: 03.02.1997 US 792031
(71) Applicant: EASTMAN KODAK COMPANY, Rochester, New York 14650 (US)
(72) Inventor: Naujokas, Andrius A., Eastman Kodak Company, Rochester, New York 14650-2201 (US)
(74) Representative: Parent, Yves

(57) **Abstract**

There is described a process for depolymerizing polyethylene naphthalate polyester to monomer components which then can be used to make virgin polyester.

## Description

This invention relates to a process for recovering ester and glycol components from polyethylene naphthalate.

Polyethylene naphthalate, which also is called polyethylene 2,6 naphthalene dicarboxylate polyesters and is abbreviated as PEN, has outstanding physical properties for a number of uses, such as in textile, film and other applications. It has been found to be particularly useful as a film base for magnetic recording materials and the photographic industry has started to use it as a film support in new photographic products. PEN, as well as its precursor dimethyl 2,6 naphthalene dicarboxylate, abbreviated as NDC, is more costly than other polyesters, such as polyethylene terephthalate, that have been used in these products. The polymer is a linear polyester produced from ethylene glycol and dimethyl 2,6 naphthalene dicarboxylate (NDC) using conventional polymerization processes. Due to relatively high cost of the polyester and to minimize the need for disposal it is desirable to recycle and reuse waste PEN.

Two distinctly different routes have been used to recycle polyesters in general. The first route is direct recycle. This can be done when the polyester scrap is completely clean or can be cleaned by washing or other means. The clean(ed) polyester is used in place of virgin polyester at the appropriate point in the manufacturing process. Depending upon the manufacturing steps in the process, degradation of the polyester can occur during such steps as melting and extrusion due to thermal and shear effects. This results in a deterioration of the physical properties of the polyester, such as a lowering of molecular weight. Generally for high grade products, such as film base, recycle polyester is blended in limited amounts with virgin polyester. Since PEN have relatively high melting and softening temperatures significant degradation of the polyester can be expected. Furthermore, if the polyester scrap is contaminated, or is not thoroughly cleaned, additional degradation of properties can occur. For applications such as photographic film and others that require high quality polyester, directly recycled material may not be suitable.

The second route is depolymerization of the polyester to component monomers, recovering the monomers and then using them to produce new polyester. This route has been used for recovery of monomers from polyethylene terephthalate polyester, abbreviated as PET. Recovery techniques are described, for example in U.S. Patents 5,414,022; 4,163,860; 3,907,868; and 3,257,335 and others.

U.S. Patent 4,163,860 discusses the use of magnesium hydroxide, as a catalyst to convert monomer to dimethyl terephthalate (DMT) in a two-step extended reaction time process at elevated temperatures. French Patent 1,081,681 presents a process where PET is first glycolized using ethylene glycol to form low molecular weight oligomers and monomer. The reaction products are then dissolved in methanol. Subsequently, sodium methylate is added to promote the reaction. These disclosures are for a process for recovery of PET.

PEN is a polycondensation polymer and, therefore, the molecular weight of the polymer can be reduced by reaction with a monohydric of dihydric alcohol at appropriate reaction conditions. The crude products then can be refined by methods such as distillation and crystallization to product starting materials of suitable purity. Recovery of polyesters utilizes the chemistry of the polyester reaction. Synthesis and depolymerization paths are controlled by equilibrium considerations due to the reversibility of the reactions.

A linear polyester is prepared by reacting a difunctional organic acid and a difunctional alcohol. In some cases it is convenient to substitute the diester of the difunctional acid (for instance dimethyl 2,6 naphthalene dicarboxylate for the naphthalene 2,6 dicarboxylic acid). Usually the reaction is carried out at elevated temperature in the presence of an appropriate catalyst. Reaction by-products are removed from the reactor to drive the equilibrium in a direction that will cause the molecular weight of the polyester to increase.

In polyester scrap recovery, selected reaction by-products are added to the reactor in order to decrease the molecular weight of the polyester by depolymerization to monomer components. The diester form of recovered monomer is preferred, since the diester is more volatile than the acid and is more readily purified.

The generic polyester reaction can be written as two steps:
Step 1: Transesterification

   Dimethyl Ester + Glycol = Half Ester + Monomer + Methanol

   CH₃-A-CH₃ + HO-G-OH = CH₃-A-G-OH + HO-G-A-G-OH + CH₃-OH
Step 2: Polycondensation

   Monomer = Polymer + Glycol

   HO-G-A-G-OH = HO-(G-A)ₓ-G-OH + HO-G-OH
wherein :
A is a difunctional acid moiety (-COO-T-COO-)
T is benzene for terephthalate and naphthalene for NDC
G is an alkylene moiety (-CH₂-CH₂- for ethylene glycol)
x is the number of repeat units in an average molecular chain, that is, the degree of polymerization.

In the transesterification step the difunctional acid can be substituted for the diester and the reaction product will be water. Likewise other alcohols may be substituted for methanol to form the diester.

Monomer has low volatility and cannot be purified by distillation. Washing and extraction however is also difficult due to poor solids crystal structure. Sometimes purification can be achieved using an appropriate adsorbent. Generally it is difficult to purify monomer. An alternative depolymerization process consists of reacting the polyester with an alcohol such as methanol to produce the diester. Reaction of PEN with methanol will produce dimethyl(2,6 naphthalene dicarboxylate) (NDC). The diester has a relatively high melting point (about 190°C), however it can be distilled at reduced pressures. Contaminants generally present in the crude NDC as well as thermal degradation during distillation make it difficult to obtain high quality materials.

A methanolysis process has been commercialized so that polyethylene terephthalate is depolymerized under ambient or relatively low pressures by passing methanol vapor through a molten mass of low molecular weight oligomers where the methanol reacts with the melt producing dimethyl terephthalate. The excess methanol vapor then strips DMT and ethylene glycol products from the melt. The process is described in U.S. patents 5,051,528; 5,298,530; 5,432,203 and others. Comparable processes have been developed for recovery of PEN polyester. Due to the significant differences in the physical properties of PET and PEN as well as their depolymerization products, known operating procedures can not be used to recover PEN using the low pressure methanolysis process.

The present invention provides a process for the depolymerizing of scrap polyethylene naphthalate polyester to monomer components that then can be used to make additional polyester.

PEN can be depolymerized to the original starting materials consisting of ethylene glycol and dimethyl naphthalene carboxylate using a low pressure methanolysis process. Temperatures of 260° to 290°C are needed to affect the depolymerization. Alcohol ratios are required in the range of 15 to 20 parts by weight to one part of NDC produced. The requirements for excess methanol can be reduced by using inert gas stripping. A relatively high quality crude NDC is produced that requires only minimal refining and enhanced conversion rates are achieved.

This process, and variations thereof, provide advantageous means for recovering monomer components from PEN polyester. The benefits and advantages of the processes will be apparent from the detailed discussion of the process below.

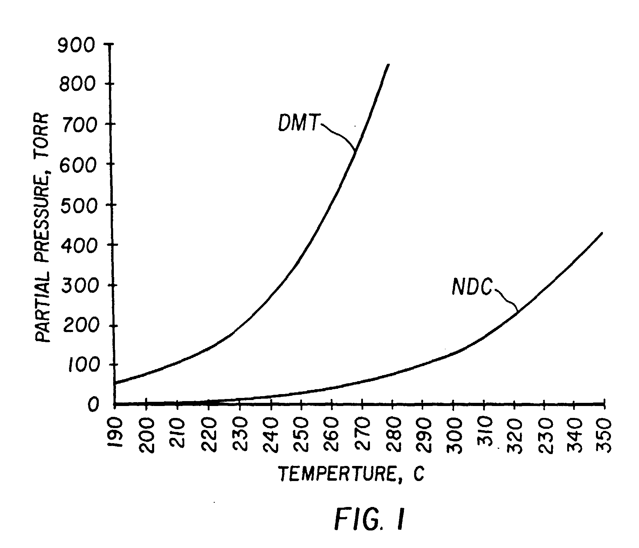

Figure 1 shows the relationship of vapor pressure and temperature for DMT and NDC.

For a better understanding of the present invention, together with other and further objects, advantages and capabilities thereof, reference is made to the following detailed description and appended claims in connection with the preceding drawings and description of some aspects of the invention.

This invention is an improvement and modification of a methanolysis recovery process for PET described in U.S. Patent 5,051,528. In this process the depolymerization is accomplished at low pressures that range from atmospheric to an elevated pressure that is still below the vapor pressure of methanol at the reaction temperatures. The methanol is generally above the critical temperature and no liquid phase is present in the reactor. The methanol vapor is bubbled through a melt of low molecular weight oligomers where the methanol reacts with the melt components. At the same time polyester scrap polymer is continuously added to the reactor at an appropriate rate. The reaction proceeds until low molecular weight and higher volatility products are formed consisting of the glycol, diester and, to some extent half ester, that are then stripped from the reactor melt by an excess of methanol vapor bubbling through the melt. The scrap feed rate is determined by the rate at which the volatile components leave the reactor. This results in a continuous steady state process. In addition, during depolymerization of the polyester scrap the process also separates certain contaminants. Low volatility contaminants as well as catalysts tend to remain in the reactor and are not carried out with the desirable reaction products of the diester and EG.

Although the polyester chemistry described above is well understood, implementing it in a practical recovery processes is not simple. Complex and sometimes interacting steps of heat and mass transfer must be considered. Some of the required processing steps include heat transfer, transport of two-phase or three-phase systems, crystallization, filtration, distillation, storage, as well as disposal of contaminants and reaction byproducts. Moreover, the physical properties of PEN and NDC differ markedly from PET and DMT. Therefore, experience in recovering DMT from PET is not directly translatable to recovery of NDC from PEN. For example, reactor design and process conditions required to recovery PEN will be different than those for PET. Physical properties of the polyester must be taken into consideration in determining suitable recovery conditions for PEN. Table 1a compares selected physical properties of the two polyesters.

**Table 1a -**

| Physical Properties of PEN and PET | | | | |
|---|---|---|---|---|
| | PET | PEN | DMT | NDC |
| Melting Point, °C | 250 | 265 | 140 | 190 |
| Tg, °C | 69 | 113 | N/A | N/A |
| Sol. in MeOH, weight % at 25°C | 0 | 0 | 1.0 | <0.1 |

The volatility of DMT and NDC are different. This impacts not only the performance of the recovery processes but also influences the ease or difficulty of refining the reaction products.

Due to the more refractory properties of PEN and NDC more harsh conditions are required to carry out the methanolysis. In order to obtain reasonably high reaction rates higher temperatures and significantly higher methanol flow rates are required than with PET.

The rate of alcohol flow and temperature of the reaction are critical to this invention. The flow rate is at superficial velocity of 2 to 10 ft./min. at standard conditions.

The embodiment of the invention is illustrated in the examples.

### Apparatus

The lab reactor system used in the experiments consisted of a 11 cm diameter, 30 cm high stainless steel reactor fitted with a three neck ground joint stainless steel reactor cover. The reactor was heated using a molten salt bath. The reaction methanol was preheated by passing liquid methanol through a coil immersed in the salt bath and then injected at the bottom of the reactor. The vapor phase products were passed through a heated line to a jacketed receiver fitted with a condenser. The products were cooled in the receiver where a slurry of solids and liquid was formed.

### Experimental Procedure

The process requires relatively low melt viscosity to operate properly. Therefore, before continuous runs could be started the startup reactor melt had to be prepared. This was accomplished by first glycolizing a sample of PEN scrap using diethylene glycol to reduce the molecular weight. The startup melt was then prepared by stripping the excess diethylene glycol using methanol vapor. This melt then was used in the depolymerization runs for PEN. In subsequent run the melt from the previous run was used for startup.

The reactor was operated until a steady state condition was achieved. This was accomplished by adjusting the scrap feed rate until a constant reactor melt volume was obtained. The reactor was then run at the chosen operating conditions for sufficient long time to assure a steady state operation.

### Example 1

The results at several operating conditions are presented in Table 1. In all cases the reactor melt depth was about 16 centimeters.

**Table 1**

| PEN Depolymerization | | |
|---|---|---|
| Temperature °C | Methanol Flow Rate ml/min | NDC Production Rate grams/hr. |
| 265 | 20 | 15 |
| 285 | 20 | 28 |
| 269 | 30 | 58 |
| 285 | 30 | 85 |

For the reactor diameter used in the experiments a 20 ml/min methanol flow rate constitutes a superficial methanol vapor velocity at standard conditions of 3.5 ft./min..

### Example 2

The PEN depolymerization was carried out using the above procedures except an inert gas consisting of nitrogen was sparged into the reactor melt. The results are listed in Table 2.

**Table 2**

| PEN Depolymerization Using Inert Gas Stripping | | | |
|---|---|---|---|
| Temperature °C Temperature | Methanol Flow | Nitrogen Flow CFH | NDC production Rate grams/hr |
| 266 | 20 | 8 | 30 |

The conditions required to depolymerize PEN show that both reaction temperatures as well as control of mass transfer rates are important. It can be seen in Table 1 that increasing methanol flow rate by one third almost triples the conversion rate. Increase in reaction temperature from about 265°to 285°C approximately doubles the conversion rate. This temperature effect is significantly higher than for PET depolymerization. Using inert gas stripping allows the reduction of methanol requirements. Introduction of nitrogen at the rates shown in Table 2 increased the conversion rate by about 100%. Nitrogen stripping will also improve conversion rates in depolymerization of PET.

The process can be operated in a batch mode by first glycolyzing the PEN scrap and then bubbling hot methanol vapor to first separate the excess glycol and then recovering the diester and free glycol fractions. Polyester scrap can also be added batchwise to the partially depleted reactor melt and the mix allowed to reach equilibrium. At this point methanol vapor can be introduced to perform the depolymerization and stripping process steps.

Methanol can also be added to the reactor as a liquid and then flashed to vapor by the hot reactor melt. Additional energy must be introduced into the reactor to vaporize the methanol.

The scrap can be fed into the reactor as solid as well as liquid streams. For instance, PEN can be introduced into reactor as solid particles of appropriate size, as a melt using an extruder or as a reduced molecular weigh melt The latter can be accomplished by reacting the scrap polymer PEN with a portion of the reactor melt, a glycol or with crude NDC. The lower viscosity feed can then be pumped into the reactor using appropriate pumps.

## Claims

1. A process for recovering monomer components from polyethylene naphthalate polyester wherein polyethylene naphthalate is depolymerized by passing alcohol vapor through a melt of oligomers of said polyester in a reactor to produce the monomers of dimethyl 2,6-naphthalene dicarboxylate and glycol which are then removed from the reactor by the stripping action of the alcohol vapor wherein the alcohol vapor is heated to a temperature of from 250° to 290°C and the monomers are produced at pressures of from atmospheric to the vapor pressure of such alcohol and the flow rate of alcohol is at superficial vapor velocity of 2 to 10 ft./min. at standard conditions.

2. The process of claim 1 wherein the alcohol is selected from the group consisting of methanol, ethanol and isopropanol.

3. The process of claim 2 wherein the alcohol is methanol.

4. The process of claim 1 wherein the temperature is 270°C.

5. The process of claim 1 wherein the oligomers have a degree of polymerization of 2 to 5.

6. The process of claim 1 wherein a gas selected from the group consisting of nitrogen, helium and carbon dioxide is added to remove the monomers from the reactors.

7. The process of claim 1 wherein the process is in a batch mode.

8. The process of claim 1 wherein the process is carried out in a continuous mode.

9. The process of claim 1 wherein the process is carried out in a semibatch mode.
